Europäisches Patentamt

European Patent Office

Office européen des brevets

(19)

(11) **EP 0 923 987 A1**

(12) # DEMANDE DE BREVET EUROPEEN

(43) Date de publication:
**23.06.1999 Bulletin 1999/25**

(51) Int Cl.$^6$: **B01J 29/068**, C07C 5/27

(21) Numéro de dépôt: **98403171.6**

(22) Date de dépôt: **16.12.1998**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **22.12.1997 FR 9716456**
**22.12.1997 FR 9716458**

(71) Demandeur: **INSTITUT FRANCAIS DU PETROLE**
**92500 Rueil Malmaison (FR)**

(72) Inventeurs:
- **Merlen, Elisabeth**
  **92500 Rueil Malmaison (FR)**
- **Alario, Fabio**
  **92200 Neuilly sur Seine (FR)**
- **Lacombe, Sylvie**
  **92500 Rueil Malmaison (FR)**
- **Benazzi, Eric**
  **78400 Chatou (FR)**
- **Joly, Jean-François**
  **69006 Lyon (FR)**

(54) **Catalyseur contenant une zéolithe EUO et son utilisation en isomérisation des composés C8 aromatiques**

(57) L'invention concerne un catalyseur comprenant au moins une zéolithe de type structural EUO, par exemple la zéolithe EU-1, au moins en partie sous forme acide, au moins une matrice, au moins un métal du groupe VIII de la classification périodique des éléments, ladite zéolithe comprenant du silicium et au moins un élément T choisi dans le groupe formé par l'aluminium, le fer, le gallium et le bore, (de préférence l'aluminium et le bore), avec un rapport Si/T atomique global supérieur à 5, ledit catalyseur étant caractérisé en ce que la dispersion dudit métal du groupe VIII est comprise entre 50% et 100% bornes incluses, et le coefficient de répartition macroscopique dudit métal du groupe VIII est compris entre 0,7 et 1,3 bornes incluses, le catalyseur présentant une résistance mécanique telle que la valeur de l'écrasement en lit est supérieure à 0,7 MPa. L'invention concerne également la préparation d'un catalyseur ainsi que son utilisation dans un procédé d'isomérisation des composés C8 aromatiques.

EP 0 923 987 A1

**Description**

L'invention concerne un catalyseur comportant au moins une zéolithe de type structural EUO, par exemple la zéolithe EU-1, au moins en partie sous forme acide, au moins une matrice (liant), au moins un élément du groupe VIII de la classification périodique des éléments, Handbook of Physics and Chemistry, 76$^{ième}$ édition, éventuellement au moins un métal appartenant à l'ensemble formé par les groupes IIIA et IVA de la classification périodique des éléments, éventuellement du soufre, ladite zéolithe comprenant du silicium et au moins un élément T choisi dans le groupe formé par l'aluminium, le fer, le gallium et le bore, de préférence l'aluminium et le bore, avec le rapport Si/T atomique global supérieur à 5, de préférence compris entre 5 et 100 bornes incluses, ledit métal du groupe VIII étant déposé de préférence sur la matrice, bien dispersé sur la surface du catalyseur et bien réparti macroscopiquement au travers du grain de catalyseur. En outre, ce catalyseur mis en forme, par exemple sous forme de billes ou d'extrudés, présente une bonne résistance mécanique.

L'invention concerne également l'utilisation du catalyseur dans un procédé d'isomérisation des composés aromatiques à 8 atomes de carbone.

L'isomérisation en xylènes de l'éthylbenzène nécessite la présence d'un métal du groupe VIII. Les formulations optimisées à base de mordénite et d'un métal du groupe VIII conduisent à des catalyseurs avec lesquels les réactions parasites restent non négligeables. Par exemple, on peut citer l'ouverture de cycles naphténiques suivie ou non de craquage ou encore les réactions de dismutation et de transalkylation des aromatiques en C8 qui conduisent à la formation d'aromatiques non recherchés. Il est donc particulièrement intéressant de trouver de nouveaux catalyseurs plus sélectifs.

Parmi les zéolithes utilisées en isoménsation des coupes C8 aromatiques, on trouve la ZSM-5, seule ou en mélange avec d'autres zéolithes comme la mordénite par exemple. Ces catalyseurs sont notamment décrits dans les brevets USP-4467129, USP-4482773 et EP-B-138617. D'autres catalyseurs sont à base de mordénite et ont été décrits par exemple dans les brevets USP-4723051, USP-4665258 et FR-A-2477903.

Le manque de sélectivité de la mordénite peut être atténué par une optimisation des formulations et/ou des traitements spécifiques comme cela a, par exemple, été décrit dans le brevet FR-2.691.914 de la demanderesse. Ces techniques permettent de diminuer les réactions parasites de dismutation.

La zéolithe EU-1 de type structural EUO, déjà décrite dans l'art antérieur, présente un réseau microporeux monodimensionnel, dont le diamètre des pores est de 4,1 x 5,7 Å (1 Å = 1 Angström = $10^{-10}$ m) (« Atlas of Zeolite Structure Types », W.M. Meier et D.H. Olson, 4$^{ème}$ Edition, 1996). D'autre part, N.A. Briscoe *et al* ont enseigné dans un article de la revue Zeolites (1988, 8, 74) que ces canaux monodimensionnels possèdent des poches latérales de profondeur 8,1 Å et de diamètre 6,8 x 5,8 Å. Le mode de synthèse de la zéolithe EU-1 et ses caractéristiques physico-chimiques ont été décrits dans le brevet EP-B1-42 226. Le brevet U.S.-4 640 829 concerne la zéolithe ZSM-50, qui, d'après l'« Atlas of Zeolite Structure Types », W.M. Meier et D.H. Olson, 4$^{ème}$ Edition, 1996, présente le même type structural EUO que la zéolithe EU-1. Ledit brevet présente un mode de synthèse de la ZSM-50 différent de celui décrit dans le brevet EP-B1-42 226 sur la zéolithe EU-1. La demande de brevet EP-A1-51 318 traite de la zéolithe TPZ-3, qui présente, d'après l' « Atlas of Zeolite Structure Types », W.M. Meier et D.H. Olson, 4$^{ème}$ Edition, 1996, le même type structural EUO que la zéolithe EU-1, et de son utilisation en tant que catalyseur contenant la zéolithe telle quelle ou mise en forme. Dans ledit document la mise en forme de la zéolithe TPZ-3 est exemplifiée par la préparation de pastilles, obtenues par pastillage d'un mélange mécanique de poudres de zéolithe et de liant. Les pastilles comprennent la zéolithe TPZ-3, un liant et éventuellement au moins un élément choisi dans le groupe formé par le fer, le cobalt, le nickel, le cuivre, le zinc, le ruthénium, le rhodium, le palladium, le rhénium, l'osmium, l'iridium et le platine, sous forme de métal ou d'oxyde de métal.

De façon surprenante, il a été découvert par la demanderesse un catalyseur mis en forme. comprenant :

- au moins une zéolithe de type structural EUO, par exemple la zéolithe EU-1, au moins en partie et de préférence pratiquement totalement sous forme acide, contenant du silicium et au moins un élément T choisi dans le groupe formé par l'lauminium, le fer, le gallium et le bore, de préférence l'aluminium et le bore, et tel que le rapport atomique global Si/T est supérieur à 5, de préférence compris entre environ 5 et 100 bornes incluses
- au moins une matrice (liant), par exemple l'alumine,
- au moins un élément du groupe VIII de la classification périodique des éléments,
- éventuellement au moins un métal appartenant à l'ensemble formé par les groupes IIIA et IVA de la classification périodique des éléments,
- et éventuellement du soufre,

ledit catalyseur étant caractérisé en ce que :

- la dispersion du ou des métaux du groupe VIII, déterminée par chimisorption, par exemple par titration $H_2$-$O_2$ ou

par chimisorption de monoxyde de carbone, est comprise entre 50% et 100% bornes incluses, de préférence entre 60% et 100% bornes incluses, et de manière encore plus préférée entre 70% et 100% bornes incluses,

- le coefficient de répartition macroscopique du(des)dit(s) métal(ux), obtenu à partir de son profil déterminé par microsonde de Castaing, défini comme le rapport des concentrations dudit métal au coeur du grain par rapport au bord de ce même grain, est compris entre 0,7 et 1,3 bornes incluses, de préférence entre 0,8 et 1,2 bornes incluses.

- la valeur de l'écrasement en lit, déterminée selon la méthode Shell (SMS 1471-74) est supérieure à 0,7 MPa.

Le catalyseur conduit à d'excellentes performances catalytiques en transformations d'hydrocarbures comme par exemple les réactions d'isomérisation de la coupe $C_8$ aromatique, c'est-à-dire de mélanges constitués de xylènes et éventuellement d'éthylbenzène.

La matrice (liant) consiste plus particulièrement en au moins un élément choisi dans le groupe formé par les argiles naturelles (comme par exemple le kaolin ou la bentonite), les argiles synthétiques, la magnésie, les alumines, les silices, les silice-alumines, l'oxyde de titane, l'oxyde de bore, la zircone, les phosphates d'aluminium, les phosphates de titane, les phosphates de zirconium, de préférence parmi les éléments du groupe formé par les alumines et les argiles.

La zéolithe de type structural EUO, par exemple la zéolithe EU-1, comprise dans le catalyseur selon l'invention, est au moins en partie, de préférence pratiquement totalement, sous forme acide, c'est-à-dire sous forme hydrogène ($H^+$), la teneur en sodium étant de préférence telle que le rapport atomique Na/T est inférieur à 0,5, de préférence inférieur à 0,1, de manière encore plus préférée inférieur à 0,02.

Le catalyseur selon l'invention contient plus particulièrement :

- en teneur pondérale, de 1 à 90% bornes incluses, et de préférence de 3 à 60% bornes incluses, et de manière encore plus préférée de 4 à 40% bornes incluses d'au moins une zéolithe de type structural EUO, par exemple la zéolithe EU-1, au moins en partie sous forme acide, comprenant du silicium et au moins un élément T choisi dans le groupe formé par l'aluminium, le fer, le gallium et le bore, de préférence l'aluminium et le bore, dont le rapport Si/T atomique global est supérieur à 5, de préférence compris entre 5 et 100 bornes incluses, de manière encore plus préférée entre 5 et 80 bornes incluses,

- au moins un élément du groupe VIII de la classification périodique des éléments, de préférence choisi dans le groupe formé par le platine et le palladium, de manière encore plus préférée le platine. La teneur pondérale du (des)dit(s) élément(s) est généralement comprise entre 0,01 et 2,0% bornes incluses, de préférence entre 0,05 et 1,0% bornes incluses. La dispersion du(des)dit(s) élément(s) du groupe VIII, déterminée par chimisorption, est comprise entre 50% et 100% bornes incluses, de préférence entre 60% et 100% bornes incluses, et de manière encore plus préférée entre 70% et 100% bornes incluses. Le coefficient de répartition macroscopique du(des)dit (s) élément(s) du groupe VIII, calculé à partir de son profil déterminé par microsonde de Castaing, ledit coefficient étant défini comme le rapport des concentrations du(des)dit(s) élément(s) du groupe VIII au coeur du grain par rapport au bord de ce même grain, est compris entre 0,7 et 1,3 bornes incluses, et de préférence entre 0,8 et 1,2 bornes incluses,

- éventuellement au moins un élément additionnel choisi dans l'ensemble formé par les groupes IIIA et IVA de la classification périodique des éléments, de préférence choisi dans le groupe formé par l'indium et l'étain. La teneur pondérale du(des)dit(s) élément(s) est généralement compnse entre 0,01 et 2,0% bornes incluses, de préférence entre 0,05 et 1,0% bornes incluses,

- éventuellement du soufre, dont la teneur est telle que le rapport du nombre d'atomes de soufre sur le nombre d'atomes de métal du groupe VIII déposés est compris entre 0,5 et bornes incluses,

- au moins une matrice, ou liant, assurant le complément à 100% dans le catalyseur.

Ledit catalyseur est en outre caractérisé en ce que la valeur d'écrasement en lit, déterminée selon la méthode Shell (SMS 1471-74) et caractérisant sa résistance mécanique est supérieure à 0,7 MPa.

Le dépôt d'au moins un élément du groupe VIII est opéré de façon telle que la dispersion du(des)dit(s) élément (s), déterminée par chimisorption se situe entre 50% et 100% bornes incluses, de préférence entre 60% et 100% bornes incluses, et de manière encore plus préférée entre 70% et 100% bornes incluses. Dans le cas où l'introduction d'au moins un élément du groupe VIII de la classification périodique des éléments et éventuellement d'au moins un élément appartenant aux groupes IIIA et IVA est effectuée après que la zéolithe de type structural EUO, par exemple la zéolithe EU-1, a été mise en forme, par exemple sous forme de billes ou d'extrudés, il est important d'obtenir une bonne répartition du(des) dit(s) élément(s) dans le catalyseur mis en forme. Cette répartition est caractérisée par son profil obtenu par microsonde de Castaing. Le rapport des concentrations de chaque élément du groupe VIII au coeur du grain par rapport au bord de ce même grain, défini comme étant le coefficient de répartition, doit être compris entre 0,7 et 1,3 bornes incluses, de préférence entre 0,8 et 1,2 bornes incluses.

L'invention concerne aussi la préparation dudit catalyseur. Pour préparer le catalyseur selon l'invention, on opère d'abord un traitement de la zéolithe de type structural EUO, par exemple la zéolithe EU-1, brute de synthèse, selon toute méthode connue de l'homme du métier, par exemple on procède à une étape de calcination sous flux d'air sec, qui a pour but d'éliminer le structurant organique occlus dans la microporosité de la zéolithe, puis on procède à au moins une étape d'échange ionique par exemple par au moins une solution de $NH_4NO_3$, de manière à éliminer au moins en partie, de préférence pratiquement totalement, tout cation alcalin, en particulier le sodium, présent en position cationique dans la zéolithe.

On poursuit la préparation du catalyseur en mélangeant la matrice et la zéolithe préparée précédemment puis on met en forme. La mise en forme du catalyseur selon l'invention est généralement telle que le catalyseur est de préférence sous forme d'extrudés ou de billes, en vue de son utilisation. Les conditions de mise en forme de la zéolithe, le choix de la matrice, éventuellement le broyage préalable de la zéolithe, le procédé de peptisation, l'ajout d'agents porogènes, le temps de malaxage, la pression d'extrusion si le catalyseur est mis sous forme d'extrudés, la vitesse et le temps de séchage, sont déterminés, pour chaque matnce, selon les règles bien connues de l'homme de métier, de manière à obtenir un catalyseur de préférence sous forme d'extrudés ou de billes.

La préparation du catalyseur se poursuit généralement par une calcination, habituellement à une température comprise entre 250°C et 600°C bornes incluses, de préférence précédée d'un séchage, par exemple à l'étuve, à une température généralement comprise entre la température ambiante et 250°C bornes incluses, de préférence entre 40°C et 200°C bornes incluses. Ladite étape de séchage est de préférence menée pendant la montée en température nécessaire pour effectuer ladite calcination.

La mise en forme de la zéolithe de type structural EUO selon l'invention, par exemple la zéolithe EU-1, peut être effectuée sur la zéolithe brute de synthèse, c'est-à-dire contenant le structurant organique et des cations alcalins, généralement le sodium. Dans ce cas l'étape de calcination sous flux d'air sec, ayant pour but d'éliminer le structurant organique, et les étapes d'échanges ioniques par au moins une solution de $NH_4NO_3$ sont réalisées sur le catalyseur mis en forme comprenant la zéolithe et la matrice.

Le catalyseur obtenu après ladite calcination et mis sous forme de billes ou d'extrudés présente des propriétés mécaniques telles que la valeur de l'écrasement en lit, déterminé selon la méthode Shell (SMS 1471-74), est supérieure à 0,7 MPa.

Le dépôt d'au moins un élément du groupe VIII de la classification périodique des éléments, et éventuellement d'au moins un élément choisi dans l'ensemble formé par les groupes IIIA et IVA de la classification périodique des éléments, peut être effectué à tout moment de la préparation, soit avant la mise en forme, soit lors du mélange de la zéolithe et de la matrice, la zéolithe étant mélangée à l'ensemble constitué par le(s) précurseur(s) du(des)dit(s) élément (s) et la matrice, soit, de manière préférée, après la mise en forme.

Lorsque l'ajout d'au moins un élément choisi dans le groupe VIII et éventuellement au moins un élément choisi dans le groupe formé par les groupes IIIA et IVA est effectué après la mise en forme, le(les)dit(s) élément(s) peut(peuvent) alors être ajouté(s), soit avant la calcination, soit, de préférence, après la calcination du mélange matrice-zéolithe. Le(les)dit(s) élément(s) ajouté(s) est(sont) généralement déposé(s), soit pratiquement totalement sur la zéolithe, soit en partie sur la zéolithe et en partie sur la matrice, soit, de préférence, pratiquement totalement sur la matrice, ceci s'effectuant, de la manière qui est connue de l'homme du métier, par le choix approprié des paramètres utilisés lors dudit dépôt, comme par exemple la nature du précurseur du(des)dit(s) élément(s). Le dépôt d'au moins un élément du groupe VIII est généralement effectué par la technique d'imprégnation à sec, d'imprégnation par excès, ou de préférence par échange(s) ionique(s). Dans le cas de l'échange ionique à partir de précurseurs à base de platine et/ ou de palladium, on utilise habituellement des sels de platine et/ou de palladium tels que l'acide hexachloroplatinique et/ou l'acide hexachloropalladique, en présence ou en absence d'agents compétiteurs, tels que par exemple l'acide chlorhydrique. Dans le cas où au moins un autre métal choisi dans l'ensemble formé par les groupes IIIA et IVA de la classification périodique des éléments est également introduit, toutes les techniques de dépôt connues de l'homme du métier et tous les précurseurs conviennent pour l'introduction de métal additionnel.

Dans le cas où le catalyseur contient plusieurs éléments du groupe VIII de la classification pénodique des éléments, les métaux peuvent être introduits, soit tous de la même façon, soit par des techniques différentes, et dans n'importe quel ordre. Dans le cas où au moins un métal choisi dans l'ensemble formé par les groupes IIIA et IVA de la classification périodique des éléments est également introduit, on peut ajouter les éléments du groupe VIII et des groupes IIIA ou IVA soit séparément soit simultanément dans au moins une étape unitaire. Lorsqu'au moins un élément des groupes IIIA ou IVA est ajouté séparément, il est préférable qu'il soit ajouté préalablement à l'élément (aux éléments) du groupe VIII. Dans le cas où la technique de dépôt utilisée est celle de l'échange ionique, plusieurs échanges successifs peuvent être nécessaires pour introduire les quantités requises de métaux.

Le platine est généralement introduit dans la matrice sous forme d'acide hexachloroplatinique, mais pour tout métal noble peuvent être également utilisés des composés ammoniaqués ou des composés tels que par exemple le chloroplatinate d'ammonium, le dichlorure de platine dicarbonyle, l'acide hexahydroxyplatinique, le chlorure de palladium, le nitrate de palladium.

L'emploi dans la présente invention d'au moins un métal noble de la famille du platine peut à titre d'exemple se faire grâce à l'utilisation de composés ammoniaqués. Dans ce cas, le métal noble sera déposé dans la zéolithe.

Dans le cas du platine, on peut citer par exemple les sels de platine II tétramines de formule $Pt(NH_3)_4X_2$, les sels de platine IV hexamines de formule $Pt(NH_3)_6X_4$; les sels de platine IV halogénopentamines de formule $(PtX(NH_3)_5)X_3$ ; les sels de platine N tétrahalogénodiamines de formule $PtX_4(NH_3)_2$ ; les complexes de platine avec les halogènes-polycétones et les composés halogénés de formule $H(Pt(acac)_2X)$ ; X étant un halogène choisi dans le groupe formé par le chlore, le fluor, le brome et l'iode , et de préférence X étant le chlore, et acac représentant le groupe $C_5H_7O_2$ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques, et les composés organiques halogénés ayant par exemple de 1 à 12 atomes de carbone par molécule. On peut citer par exemple le n-heptane, le méthylcyclohexane, le toluène et le chloroforme. On peut aussi utiliser les mélanges de solvants.

Le métal additionnel, éventuellement introduit en plus, choisi dans le groupe formé par les éléments des groupes IIIA et IVA, peut être introduit par l'intermédiaire de composés tels que par exemple les chlorures, les bromures et les nitrates, les alkyls d'éléments des groupes IIIA et IV, soit par exemple l'étain et l'indium, les alkyl étain, le nitrate et le chlorure d'indium.

Si ce métal est introduit avant le métal noble, le composé du métal utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal noble, on procède à une calcination sous air.

Ce métal peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés du métal, on peut citer en particulier le tétrabutylétain dans le cas de l'étain, et le triphénylindium dans le cas de l'indium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer par exemple le n-heptane, le méthylcyclohexane et le chloroforme. On peut aussi utiliser des mélanges des solvants définis ci-dessus.

Le dépôt d'au moins un élément du groupe VIII et éventuellement d'au moins un élément des groupes IIIA ou IVA est suivi de préférence d'une calcination sous air ou oxygène, généralement entre 250° C et 600°C bornes incluses, de préférence entre 350°C et 550°C bornes incluses, et pour une durée comprise entre 0,5 et 10 heure(s) bornes incluses, de préférence entre 1 et 4 heure(s) bornes incluses. On procède ensuite éventuellement à une réduction sous hydrogène, généralement à une température comprise entre 300 et 600°C, de préférence entre 350°C et 550°C bornes incluses, et pour une durée comprise entre 1 et 10 heure(s) bornes incluses, de préférence entre 2 et 5 heures bornes incluses, de façon à obtenir le(les)dit(s) élément(s) principalement sous forme réduite nécessaire à l'activité catalytique.

Par exemple, une des méthodes préférées de préparation du catalyseur selon l'invention consiste à malaxer au moins une zéolithe de type structural EUO, par exemple la zéolithe EU-1, dans un gel humide de matrice (obtenu généralement par mélange d'au moins un acide et d'une poudre de matrice), par exemple d'alumine, pendant une durée nécessaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm bornes incluses, de préférence entre 0,4 et 2,5 mm bornes incluses et de préférence encore entre 0.8 et 2,0 mm bornes incluses. Puis, après séchage pendant quelques heures à environ 120°C en étuve et après calcination, par exemple pendant environ 2 heures à environ 400°C, le ou les éléments du groupe VIII et éventuellement le ou les éléments des groupes IIIA et IVA, par exemple le platine, sont déposés, par exemple par échange ionique, avec par exemple de l'acide hexachloroplatinique en présence d'un agent compétiteur (par exemple l'acide chlorhydrique) ledit dépôt étant suivi d'une calcination, par exemple pendant environ heures à environ 400°C.

Dans le cas où le catalyseur de la présente invention contient du soufre, le soufre est introduit sur le catalyseur mis en forme, calciné, contenant le ou les élément(s) cité(s) précédemment, soit *in situ* avant la réaction catalytique, soit *ex situ.* La sulfuration s'effectue en utilisant tout agent sulfurant bien connu de l'homme de métier, tel que par exemple le disulfure de diméthyle ou le sulfure d'hydrogène. La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration.

Le catalyseur selon l'invention présente, outre une excellente résistance mécanique à l'écrasement, d'excellentes performances catalytiques en transformations d'hydrocarbures, comme par exemple l'isomérisation des composés aromatiques à 8 atomes de carbone, c'est-à-dire de mélanges constitués de xylènes et éventuellement d'éthylbenzène.

Ainsi, l'invention concerne également un procédé d'isomérisation d'une coupe C8 aromatiques en présence du catalyseur selon l'invention.

Ledit procédé est mis en oeuvre généralement selon les conditions opératoires suivantes :

- une température comprise entre 300°C et 500°C bornes incluses. de préférence entre 320°C et 450°C bornes incluses et de manière encore plus préférée entre 340°C et 430°C bornes incluses,
- une pression partielle d'hydrogène comprise entre 0,3 et 1,5 MPa bornes incluses, de préférence entre 0,4 et 1,2 MPa bornes incluses et de manière encore préférée entre 0,7 et 1,2 MPa bornes incluses,
- une pression totale comprise entre 0,45 et 1,9 MPa bornes incluses, de préférence entre 0,6 et 1,5 MPa bornes incluses,
- une vitesse spatiale d'alimentation, exprimée en kilogramme de charge introduite par kilogramme de catalyseur et par heure, comprise entre 0,25 et $30h^{-1}$ bornes incluses, de préférence entre 1 et $10h^{-1}$ bornes incluses, et de manière encore préférée entre et 6 $h^{-1}$ bornes incluses.

Les exemples suivants illustrent l'invention sans toutefois en limiter la portée.

**Exemple 1: Préparation du catalyseur C1 contenant en poids 10,0% de zéolithe EU-1 de rapport Si/A1 égal à 18,3, 89,7% d'alumine et 0,29% de platine.**

La matière première utilisée est une zéolithe EU-1, brute de synthèse, comprenant le structurant organique, du silicium et de l'aluminium, possédant un rapport atomique Si/A1 global égal à 13,6 , une teneur pondérale en sodium par rapport au poids en zéolithe EU-1 sèche d'environ 1,5%, correspondant à un rapport atomique Na/A1 de 0,6.

Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.

A l'issue de ces traitements, la zéolithe EU-1 sous forme $NH_4$ a un rapport Si/A1 atomique global égal à 18,3 , une teneur pondérale en sodium par rapport au poids de zéolithe EU-1 sèche de 50 ppm, correspondant à un rapport atomique Na/A1 de 0,003, une surface spécifique mesurée par la méthode BET de 407 $m^2$/g et un volume poreux, à l'azote, mesuré à -196°C et à $P/P_0$ = 0,15, de 0,16 $cm^3$ d'azote liquide par gramme.

La zéolithe EU-1 est ensuite mise en forme par extrusion avec un gel d'alumine de manière à obtenir, après séchage et calcination sous air sec, le support constitué d'extrudés de 1,4 mm de diamètre, qui contient en poids 10 % de zéolithe EU-1 sous forme H et 90 % d'alumine. Le diamètre des pores du catalyseur ainsi préparé, mesuré par porosimétrie au mercure, se situe entre 40 et 90 Å, la distribution des diamètres de ces mésopores étant monomodale et centrée sur 70 Å. La valeur de l'écrasement lit, obtenu selon la méthode Shell, est de 1,1 MPa.

Le support ainsi obtenu est soumis à un échange anionique avec de l'acide hexachloroplatinique en présence d'un agent compétiteur (acide chlorhydrique), de manière à introduire 0,3% poids de platine par rapport au catalyseur. Le solide humide est ensuite séché à 120°C pendant 12 heures et calciné sous un débit d'air sec à la température de 500°C pendant une heure.

Le catalyseur C1 ainsi obtenu contient en poids 10,0% de zéolithe EU-1 sous forme H, 89,7% d'alumine et 0,29% de platine. Il présente, pour la phase métallique, une dispersion de 95%, déterminée par chimisorption, et un coefficient de répartition du platine de 0,90, déterminé par microsonde de Castaing.

**Exemple 2 : Préparation du catalyseur C2 contenant en poids 10,0% de zéolithe EU-1 de rapport Si/A1 égal à 31, 89,7% d'alumine et 0,28% de platine.**

La matière première utilisée est une zéolithe de type structural EUO, la zéolithe EU-1, brute de synthèse, comprenant le structurant organique, du silicium et de l'aluminium, possédant un rapport atomique Si/A1 global égal à 28, une teneur pondérale en sodium par rapport au poids en zéolithe EU-1 sèche d'environ 0,4 %, correspondant à un rapport atomique Na/A1 de 0,30.

Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.

A l'issue de ces traitements, la zéolithe EU-1 sous forme $NH_4$ a un rapport Si/A1 atomique global égal à 31, une teneur pondérale en sodium par rapport au poids de zéolithe EU-1 sèche de 100 ppm, correspondant à un rapport atqmique Na/A1 de 0,008, une surface mesurée par la méthode BET de 435 $m^2$/g et un volume poreux, à l'azote, mesuré à -196°C et à $P/P_0$ = 0,15, de 0,18 $cm^3$ d'azote liquide par gramme.

La zéolithe EU-1 est ensuite mise en forme par extrusion avec un gel d'alumine de manière à obtenir, après séchage et calcination sous air sec, le support constitué d'extrudés de 1,4 mm de diamètre, qui contient en poids 10 % de zéolithe EU-1 sous forme H et 90 % d'alumine. Le diamètre des pores du catalyseur ainsi préparé, mesuré par porosimétrie à mercure, se situe entre 100 et 1000 Å, la distribution des diamètres de ces mésopores étant monomodale et centrée sur 330 Å. Cette différence de porosité entre les catalyseurs C1 et C2 résulte de l'utilisation de gels d'alumine différents. La valeur de l'écrasement lit, obtenu selon la méthode Shell, est de 1,0 MPa.

Le support ainsi obtenu est soumis à un échange anionique avec de l'acide hexachloroplatinique en présence d'un agent compétiteur (acide chlorhydrique), de manière à introduire 0,3% poids de platine par rapport au catalyseur. Le solide humide est ensuite séché à 120°C pendant 12 heures et calciné sous un débit d'air sec à la température de 500°C pendant une heure.

Le catalyseur C2 ainsi obtenu contient en poids 10,0% de zéolithe EU-1 sous forme H, 89,7% d'alumine et 0,28% de platine. Il présente, pour la phase métallique, une dispersion de 94%, déterminée par chimisorption, et un coefficient de répartition du platine de 0,92, déterminé par microsonde de Castaing.

**Exemple 3 : Préparation du catalyseur C3 contenant en poids 29,9% de zéolithe EU-1 de rapport Si/A1 voisin de 44, 69,8% d'alumine et 0,29% de platine.**

La matière première utilisée est une zéolithe de type structural EUO, la zéolithe EU-1, brute de synthèse, comprenant le structurant organique, du silicium et de l'aluminium, possédant un rapport atomique Si/A1 global égal à environ 44, une teneur pondérale en sodium par rapport au poids en zéolithe EU-1 sèche d'environ 0,5 %, correspondant à un rapport atomique Na/A1 proche de 0,6.

Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.

A l'issue de ces traitements, la zéolithe EU-1 sous forme $NH_4$ a un rapport Si/A1 atomique global proche de 44, une teneur pondérale en sodium par rapport au poids de zéolithe EU-1 sèche de 100 ppm, correspondant à un rapport atomique Na/A1 d'environ 0,012 %, une surface mesurée par la méthode BET de 420 $m^2$/g et un volume poreux, à l'azote, mesuré à -196°C et à $P/P_0 = 0,15$, de 0,17 $cm^3$ d'azote liquide par gramme.

La zéolithe EU-1 est ensuite mise en forme par extrusion avec un gel d'alumine de manière à obtenir, après séchage et calcination sous air sec, le support constitué d'extrudés de 1,4 mm de diamètre, qui contient en poids 30 % de zéolithe EU-1 sous forme H et 70 % d'alumine. Le diamètre des pores du catalyseur ainsi préparé, mesuré par porosimétrie à mercure, se situe entre 40 et 90 Å, la distribution des diamètres de ces mésopores étant monomodale et centrée sur 70 Å. La valeur de l'écrasement lit, obtenu selon la méthode Shell, est de 0,89 MPa.

Le support ainsi obtenu est soumis à un échange anionique avec de l'acide hexachloroplatinique en présence d'un agent compétiteur (acide chlorhydrique), de manière à introduire 0,3% poids de platine par rapport au catalyseur. Le solide humide est ensuite séché à 120°C pendant 12 heures et calciné sous un débit d'air sec à la température de 500°C pendant une heure.

Le catalyseur C3 ainsi obtenu contient en poids 29,9% de zéolithe EU-1, 69,8% d'alumine et 0,29% de platine. Il présente, pour la phase métallique, une dispersion de 92%, déterminée par chimisorption, et un coefficient de répartition du platine de 0,94, déterminé par microsonde de Castaing.

**Exemple 4 : Préparation du catalyseur C4 contenant en poids 10,0% de zéolithe EU-1 de rapport Si/A1 égal à 18,3, 89,6% d'alumine, 0,28% de platine et 0,14% d'étain.**

Dans la préparation du catalyseur C4, des dépôts d'étain puis de platine sont effectués sur le support obtenu dans l'exemple 1.

L'étain est d'abord déposé sur ce solide par échange ionique avec une solution de chlorure d'étain $SnCl_2$ en présence d'un agent compétiteur (acide chlorhydrique), de manière à obtenir 0,15 % poids d'étain par rapport au catalyseur. Ce dépôt est suivi d'une calcination. On procède ensuite à un deuxième échange anionique avec de l'acide hexachloroplatinique en présence d'un agent compétiteur (acide chlorhydrique), de manière à introduire 0,3% poids de platine par rapport au catalyseur. Le solide humide est ensuite séché à 120°C pendant 12 heures et calciné sous un débit d'air sec à la température de 500°C pendant une heure.

Le catalyseur C4 ainsi obtenu contient en poids 10,0% de zéolithe, 89,6% d'alumine, 0,28% de platine et 0,14% d'étain. Il présente, pour la phase métallique, une dispersion de 91%, déterminée par chimisorption, et un coefficient de répartition du platine de 0,89, déterminé par microsonde de Castaing. La résistance à l'écrasement du catalyseur C4 est la même que celle mesurée sur le catalyseur C1.

**Exemple 5 : Préparation du catalyseur C5 non conforme à l'invention, contenant de la mordénite et 0,3 % poids de platine.**

La zéolithe de départ est une mordénite de rapport Si/Al = 5,2 et de volume de maille élémentaire de 2,794 $nm^3$. La zéolithe est soumise à trois échanges ioniques dans une solution $NH_4NO_3$ 10N à environ 100°C pendant 4 heures. Le solide ainsi obtenu contient 25 ppm de sodium.

Cette zéolithe est ensuite mise en forme par extrusion (diamètre d'extrusion = 1,4 mm) avec un gel d'alumine de manière à obtenir, après séchage et calcination sous air sec, un support qui contient en poids 10 % de zéolithe mordénite forme hydrogène et 90 % d'alumine.

Ce support est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur C5 ainsi obtenu contient en poids, 10,0 % de mordénite forme hydrogène, 89,7 % d'alumine et 0,3 % de platine. La dispersion du platine, mesurée par chimisorption, est de 95 % et le coefficient de répartition du platine déterminé par microsonde de Castaing est de 0,95. Le diamètre des pores, mesuré par porosimétrie au mercure, se situe entre 40 et 90 Å, la distribution des diamètres de ces mésopores étant monomodale et centrée sur 70 Å. La valeur de l'écrasement lit, obtenu selon la méthode Shell, est de 1,5 MPa.

**Exemple 6 : Préparation du catalyseur C6 non conforme à l'invention, contenant de la zéolithe EU-1 et 0,3 % poids de platine.**

Le catalyseur C6 est préparé selon le même protocole que le catalyseur C1 mais la dernière étape de calcination à 500°C est supprimée et la préparation se termine donc par un simple séchage à 120°C.

La mesure de la dispersion métallique par chimisorption d'oxygène conduisait, sur le catalyseur C1, à une valeur de 95 %, tandis que l'on n'obtient que 43 % pour le catalyseur C6, non conforme à l'invention.

**Exemple 7 : Préparation du catalyseur C7 non conforme à l'invention, contenant de la zéolithe EU-1 et 0,3 % poids de platine.**

Le catalyseur C7 est obtenu par pastillage d'un mélange de zéolithe EU-1 telle qu'elle est décrite dans l'exemple 1 et d'une alumine sur laquelle 0,33 % poids de platine a été préalablement déposé.

Le platine est déposé sur cette alumine par échange anionique avec de l'acide hexachloroplatinique en présence d'un agent compétiteur (acide chlorhydrique). L'alumine humide est ensuite séchée à 120°C pendant 12 heures et calcinée sous débit d'air sec à la température de 500°C pendant une heure. La phase métallique présente une dispersion de 99 % mesurée par chimisorption d'oxygène. On procède ensuite à la mise en forme par pastillage.

Le catalyseur pastillé C7 ainsi obtenu contient en poids, 10,0 % de zéolithe EU-1 forme hydrogène, 89,7 % d'alumine et 0,3 % de platine.

La principale différence entre les catalyseurs C1 et C7 réside donc dans la mise en forme qui n'est pas conforme dans le cas du catalyseur C7. On obtient dans ce cas une valeur d'écrasement Shell de 0,3 MPa qui est nettement inférieure à celle de l'échantillon C1.

**Exemple 8 : Evaluation des propriétés catalytiques des catalyseurs C1 à C7 en isomérisation d'une coupe C8 aromatique, avec 5 g de catalyseur.**

Les performances des catalyseurs C1 à C7 ont été évaluées dans l'isomérisation d'une coupe C8 aromatique contenant principalement du méta-xylène, de l'ortho-xylène et de l'éthylbenzène avec 5 g de catalyseur. Les conditions opératoires sont les suivantes :

- température : 390°C,
- pression totale : 15 bar, (1 bar = 0,1 MPa)
- pression partielle d'hydrogène : 12 bar.

Les catalyseurs sont préalablement traités avec une charge contenant du disulfure de diméthyle (DMDS) en présence d'hydrogène, avec une concentration telle que le rapport atomique soufre/métal soit de 1,5 sauf pour le catalyseur C4. Le catalyseur est ensuite maintenu pendant 3 heures à 400°C sous débit d'hydrogène puis la charge est injectée.

Les catalyseurs ont été comparés en terme d'activité (par les approches à l'équilibre du para-xylène et de l'éthylbenzène, et par la conversion de l'éthylbenzène), et en terme de sélectivité par les pertes nettes à iso-approche à l'équilibre du para-xylène.

De plus, les réactions parasites conduisent à trois types de pertes : les pertes vers les paraffines résultant essentiellement de réactions d'ouverture de cycles naphténiques suivies de craquage, les pertes vers les aromatiques formés par réactions de dismutation et de transalkylation des aromatiques à 8 atomes de C AC8, et enfin les pertes vers les naphtènes dont les naphtènes à 8 atomes de carbone (N8) dus à l'hydrogénation des aromatiques. Les N8 pouvant être recyclés, on comparera les pertes par craquage et dismutation/transalkylation incluant les naphtènes autres que N8 (dont la somme constitue les pertes nettes) en prenant une base 100, pour chacune de ces pertes, pour le catalyseur A non conforme à l'invention.

Pour le calcul des approches à l'équilibre (AEQ), les concentrations en éthylbenzène (%EB) sont exprimées par rapport aux quatre isomères AC8, et celles en para-xylène (%pX) par rapport aux trois isomères xylènes.

Les approches à l'équilibre (AEQ) sont définies de la manière suivante :

$$pX\ AEQ\ (\%) = 100 \times (\%pX_{effluent} - \%pX_{charge}) / (\%pX_{équilibre} - \%pX_{charge})$$

$$EB\ AEQ\ (\%) = 100 \times (\%EB_{effluent} - \%EB_{charge}) / (\%EB_{équilibre} - \%EB_{charge})$$

Les pertes par craquage (P1) sont des pertes en AC8 sous forme de paraffines (PAR) de C1 à C8 :

$$P1(\%poids) = 100 \times [(\%PAR_{effluent}\ x\ poids\ d'effluent) - (\%PAR_{charge}\ x\ poids\ de\ charge)] / (\%AC8_{charge}\ x\ poids\ de\ charge)$$

Les pertes par dismutation/transalkylation (P2) sont des pertes en AC8 sous forme de naphtènes autres que N8, de toluène, de benzène et de C9+ aromatiques (OAN):

$$P2(\%poids) = 100 \times [(\%OAN_{effluent}\ x\ poids\ d'effluent) - (\%OAN_{charge}\ x\ poids\ de\ charge)] / (\%AC8_{charge}\ x\ poids\ de\ charge)$$

La somme des pertes P1 et P2 représente les pertes nettes.

Les données présentées dans le tableau 1 ont été obtenues à iso-conditions expérimentales.

Tableau 1

| Catalyseurs | C1 | C2 | C3 | C5 non conforme | C6 non conforme | C7 non conforme |
|---|---|---|---|---|---|---|
| Si/Al | 18,3 | 31 | 44 | MOR | 18,3 | 18,3 |
| Teneur en zéolithe (%) | 10 | 10 | 30 | 10 | 10 | 10 |
| pX AEQ (%) | 98,0 | 97,1 | 97,8 | 94,5 | 97,7 | 97,9 |
| EB AEQ (%) | 90,8 | 81,2 | 87,7 | 86,2 | 66,1 | 88,8 |
| conversion EB (%) | 55,9 | 52,2 | 55,4 | 54,1 | 44,1 | 59,8 |
| pertes nettes (% poids) | 5,7 | 5,2 | 7,1 | 6,7 | 6,3 | 14,9 |

On constate d'après les résultats du tableau 1 que les catalyseurs C1 à C3 conformes à l'invention sont beaucoup plus actifs que le catalyseur C5 non conforme, puisqu'ils conduisent à iso-conditions opératoires à une pX AEQ de 98,0 %, 97,1 % et 97,8 % respectivement (contre 94,5 % pour le catalyseur C5).

De plus, les catalyseurs selon l'invention C1 à C3 présentent une conversion de l'éthylbenzène de 55,9 %, 52,2 % et 55,4 % respectivement, donc bien plus élevée que dans le cas du catalyseur non conforme C6 (44,1 %). Enfin, les catalyseurs C1 à C3 présentent des pertes nettes de 5,7 %, 5,2 % et 7,1 % respectivement, donc beaucoup plus faibles que dans le cas du catalyseur C7 non conforme (14,9 %) qui est donc beaucoup moins sélectif.

Par ailleurs, ces catalyseurs ont été comparés à iso pX AEQ (environ 95,5%) en faisant varier les débits massiques de charge. Ces résultats sont présentés dans le tableau 2.

Tableau 2

| Catalyseurs | C1 | C2 | C3 | C4 | C5 non conforme |
|---|---|---|---|---|---|
| Si/Al | 18,3 | 31 | 44 | 18,3 | MOR |
| Teneur en zéolithe (%) | 10 | 10 | 30 | 10 | 10 |
| pX AEQ (%) | 95.5 | 94,5 | 95,3 | 94,8 | 94.5 |
| pertes nettes (% poids) | 4,7 | 4,5 | 5,9 | 4,4 | 6,7 |
| craquage | 98 | 107 | 103 | 86 | 100 |
| dismutation/transalkylation | 53,6 | 43,3 | 80,4 | 56,9 | 100 |

A iso pX AEQ, le tableau 2 fait apparaître que les catalyseurs C1 à C4 conformes à l'invention sont également beaucoup plus sélectifs que le catalyseur C5 non conforme. En effet, pour une pX AEQ de 95,5 % environ, les pertes nettes sont respectivement de 4,7%, 4,5 %, 5,9 % et 4,4 % poids pour les catalyseurs C1 à C4 contre 6,7 % poids pour le catalyseur C5. Ce gain très important dans le cas des catalyseurs conformes à l'invention, se retrouve au niveau des pertes par dismutation/transalkylation. L'activité et la sélectivité obtenues lors de l'utilisation des catalyseurs selon l'invention, à base d'une zéolithe de structure EUO, en isomérisation d'une coupe C8 aromatique sont donc nettement améliorées par rapport à l'art antérieur.

Par ailleurs, des tests de stabilité ont été effectués. Les catalyseurs C1 et C6 ont été testés dans les conditions décrites précédemment durant 800 heures. Ils ont ensuite été déchargés et régénérés dans les mêmes conditions : leurs références sont alors respectivement C1R et C6R. La régénération consiste à pratiquer un traitement sous air à une température de 500°C de manière à brûler le coke déposé sur le catalyseur au cours des 800 heures de réaction. A l'issue de cette régénération, un second test de 800 heures sous charge a été effectué sur les catalyseurs C1R et C6R, dans des conditions opératoires identiques à celles du premier test de 800 heures. Les résultats sont présentés dans le tableau 3.

Tableau 3

| Catalyseur | C1 (conforme) | C6 (non conforme) |
|---|---|---|
| conversion EB (%) à t=36 h | 55,9 | 44,1 |
| conversion EB (%) à t=800 h | 53,2 | 39,4 |
| chute de conversion EB (%) | 4,9 | 10,6 |
| Catalyseur | C1R (conforme) | C6R (non conforme) |
| conversion EB (%) à t=36 h | 55,0 | 41,9 |
| conversion EB (%) à t=800 h | 51,7 | 36,8 |
| chute de conversion EB (%) | 6,0 | 12,2 |

Le catalyseur C1 conforme à l'invention présente une désactivation (mesurée au travers de la chute de conversion de l'éthylbenzène) de 4,9 % pour 800 heures de test. La régénération permet de retrouver, pour le catalyseur C1, une conversion de l'éthylbenzène de 55,0 % contre 55,9 % pour le catalyseur frais. Le catalyseur C6 non conforme est beaucoup moins stable avec 10,6 % de désactivation pour un même nombre d'heures sous charge. La régénération est également moins efficace.

A travers cet exemple, il apparaît que le catalyseur C1 conforme à l'invention, pour lequel le métal du groupe VIII est bien dispersé à la surface du catalyseur, est plus actif et plus stable que le catalyseur C6 non conforme.

**Exemple 9 : Evaluation des propriétés catalytiques des catalyseurs C1 et C5 en isomérisation d'une coupe C8 aromatique, avec 60 g de catalyseur.**

Les performances des catalyseurs C1 et C5 ont été évaluées dans l'isomérisation d'une coupe C8 aromatique contenant principalement du méta-xylène, de l'ortho-xylène et de l'éthylbenzène avec 60 g de catalyseur. Les conditions opératoires sont les suivantes :

- température : 375°C,
- pression totale : 9 bar, (1 bar = 0,1 MPa)
- $H_2$/HC : 4

Les résultats obtenus à iso-pX AEQ sont présentés dans le tableau 4.

Tableau 4

| Catalyseurs | C1 | C5 |
|---|---|---|
| Si/Al | 18,3 | MOR |
| Teneur en zéolithe (%) | 10 | 10 |
| pph ($h^{-1}$) | 4 | 2,51 |
| pX AEQ (%) | 93,1 | 92,91 |
| conversion EB (%) | 41,0 | 46,1 |
| pertes nettes (%) | 1,6 | 4,8 |
| pertes craquage | 69 | 100 |
| dismutation/transalkylation | 20 | 100 |

On constate d'après le tableau 4 que le catalyseur C1 selon l'invention est beaucoup plus sélectif que le catalyseur C5 non conforme puisque les pertes nettes sont de 1,6 % pour C1 contre 4,8 % pour C5.
De plus, le catalyseur selon l'invention est plus actif puisqu'à iso-pX AEQ, on travaille à une pph de 4 $h^{-1}$ contre un pph de 2,51 $h^{-1}$ pour C5.

## Revendications

1. Catalyseur comprenant au moins une zéolithe de type structural EUO, au moins en partie sous forme acide, au moins une matnce, au moins un métal du groupe VIII de la classification pénodique des éléments, ladite zéolithe comprenant du silicium et au moins un élément T choisi dans le groupe formé par l'aluminium, le fer, le gallium et le bore, avec un rapport Si/T atomique global supérieur à 5, ledit catalyseur étant caractérisé en ce que la dispersion dudit métal du groupe VIII est comprise entre 50% et 100% bornes incluses, et le coefficient de répartition macroscopique dudit métal du groupe VIII est compris entre 0,7 et 1,3 bornes incluses, le catalyseur présentant une résistance mécanique telle que la valeur de l'écrasement en lit est supérieur à 0,7 MPa..

2. Catalyseur selon la revendication 1 caractérisé en ce que la dispersion du métal du groupe VIII est comprise entre 60 et 100%, et de préférence entre 70 et 100%.

3. Catalyseur selon l'une des revendications 1 à 2 caractérisé en ce que le coefficient de répartition macroscopique est compris entre 0,8 et 1,2.

4. Catalyseur selon l'une des revendications 1 à 3 caractérisé en ce qu'il est mis en forme sous forme de billes ou d'extrudés.

5. Catalyseur selon l'une des revendications 1 à 4 caractérisé en ce que la zéolithe de type structural EUO est la zéolithe EU-1.

6. Catalyseur selon l'une des revendications 1 à 5 caractérisé en ce que l'élément T est choisi dans le groupe formé par l'aluminium et le bore.

7. Catalyseur selon l'une des revendications 1 à 6 caractérisé en ce que la matrice est l'alumine

8. Catalyseur selon l'une des revendications 1 à 7 caractérisé en ce que le métal du groupe VIII est choisi dans le groupe formé par le platine et le palladium.

9. Catalyseur selon l'une des revendications 1 à 8 caractérisé en ce que la zéolithe est au moins en partie sous forme acide avec un rapport atomique Na/T inférieur à 0,5.

10. Catalyseur selon l'une des revendications 1 à 9 caracténsé.en ce qu'il contient de 1% à 90% en poids d'au moins une zéolithe de type structural EUO, de préférence de 3 à 60% et de manière encore plus préférée de 4 à 40%.

11. Catalyseur selon l'une des revendications 1 à 10 caractérisé en ce que la teneur pondérale du ou des métaux du groupe VIII est comprise entre 0,01% et 2,0%, de préférence entre 0,05 et 1,0% par rapport au poids total du catalyseur.

12. Catalyseur selon l'une des revendications 1 à 11 caractérisé en ce qu'il comprend en outre au moins un élément choisi dans le groupe formé par les groupes IIIA et IVA de la classification pénodique des éléments.

13. Catalyseur selon l'une des revendications 1 à 12 caracténsé en ce que l'élément choisi dans le groupe formé par les groupes IIIA et IVA de la classification périodique est l'étain et/ou l'indium.

14. Catalyseur selon l'une des revendications 1 à 13 caractérisé en ce que la teneur d'au moins un élément choisi dans le groupe formé par les groupes IIIA et IVA de la classification périodique des éléments est comprise entre 0,01% et 2,0%, de préférence entre 0,05 et 1% par rapport au catalyseur.

15. Catalyseur selon l'une des revendications 1 à 14 caracténsé en ce qu'il comprend du soufre en une teneur telle que le rapport du nombre d'atomes de soufre sur le nombre d'atomes de métal du groupe VIII déposés est compris entre 0,5 et 2.

16. Procédé de préparation du catalyseur selon l'une des revendications 1 à 15 caractérisé en ce qu'il comprend une étape de traitement de la zéolithe de type structural EUO brute de synthèse, une étape dans laquelle la matrice et ladite zéolithe sont mélangées puis ledit mélange est mis en forme, une étape de calcination généralement effectuée à une température comprises entre 250°C et 600°C, bornes incluses, le dépôt d'au moins un métal du groupe VIII pouvant être effectué à tout moment de la préparation.

17. Procédé de préparation du catalyseur selon la revendication 16 tel que le dépôt du métal du groupe VIII est suivi d'une calcination effectuée à une température comprise entre 250 et 600°C.

18. Procédé selon l'une des revendications 16 à 17 caractérisé en ce que le métal du groupe VIII est déposé après l'étape de calcination qui suit la mise en forme du mélange matrice-zéolithe.

19. Procédé selon l'une des revendications 16 à 18 caracténsé en ce que le métal du groupe VIII est déposé à plus de 90% totalement sur le liant.

20. Procédé selon l'une des revendications 16 à 19 caractérisé en ce qu'il comprend le dépôt d'au moins un élément choisi dans le groupe formé par les élément des groupes IIIA et IVA, ce dépôt pouvant être effectué à tout moment de la préparation.

21. Procédé selon l'une des revendications 16 à 20 caractérisé en ce que le dépôt d'au moins un élément choisi dans le groupe formé par les éléments des groupes IIIA et IVA est effectué avant le dépôt du métal du groupe VIII.

22. Procédé selon l'une des revendications 16 à 21 caractérisé en ce que le soufre est introduit sur le catalyseur mis en forme, calciné et réduit, contenant le ou les éléments déposés, soit in situ avant la réaction catalytique, soit ex situ.

23. Utilisation d'un catalyseur selon l'une des revendications 1 à 15 ou préparé selon l'une des revendications 16 à 22 dans un procédé d'isoménsation d'une charge comprenant des composés aromatiques à 8 atomes de carbone par molécule.

24. Utilisation d'un catalyseur selon la revendication 23 caractérisé en ce que la charge est choisie parmi un mélange de xylènes, de l'éthylbenzène, un mélange de xylènes et de l'éthylbenzène.

25. Utilisation d'un catalyseur selon l'une des revendications 23 à 24 caractérisé en ce qu'il est mis en oeuvre à une température comprise entre 300 et 500°C, avec une pression partielle d'hydrogène comprise entre 0,3 et 1,5 MPa, avec une pression totale comprise entre 0,45 et 1,9 MPa et une vitesse spatiale d'alimentation comprise entre 0,25 et 30 h$^{-1}$.

**Office européen des brevets**

## RAPPORT DE RECHERCHE EUROPEENNE

Numéro de la demande

EP 98 40 3171

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | US 4 593 138 A (CASCI JOHN L ET AL) 3 juin 1986<br>* colonne 6, ligne 28 - ligne 31 *<br>* colonne 6, ligne 55 - ligne 65 *<br>* revendication 6 *<br>& US 4 537 754 A (CASCI ET AL.) 27 août 1985<br>& EP 0 042 226 A (ICI PLC) 23 décembre 1981 | 1,4-14 | B01J29/068<br>C07C5/27 |
| X | EP 0 055 045 A (ICI PLC) 30 juin 1982<br>* page 3, ligne 34 *<br>* page 4, ligne 5 - ligne 10 * | 1,5 | |
| A | EP 0 537 389 A (COUNCIL SCIENT IND RES) 21 avril 1993<br>* abrégé * | 1 | |
| A | US 4 741 891 A (LOWE BARRIE M ET AL) 3 mai 1988<br>* colonne 4, ligne 20 - ligne 46 * | 1 | DOMAINES TECHNIQUES RECHERCHES (Int.Cl.6) |
| A,D | EP 0 051 318 A (TEIJIN PETROCHEM INDUSTRIES) 12 mai 1982<br>* revendications * | 23 | B01J<br>C07C |
| A | WO 96 16004 A (EXXON CHEMICAL PATENTS INC) 30 mai 1996<br>* revendications * | 23 | |

Le présent rapport a été établi pour toutes les revendications

| Lieu de la recherche | Date d'achèvement de la recherche | Examinateur |
|---|---|---|
| LA HAYE | 30 mars 1999 | Van Geyt, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 98 40 3171

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

30-03-1999

| Document brevet cité au rapport de recherche | | Date de publication | Membre(s) de la famille de brevet(s) | | Date de publication |
|---|---|---|---|---|---|
| US 4593138 | A | 03-06-1986 | EP | 0042226 A | 23-12-1981 |
| | | | US | 4537754 A | 27-08-1985 |
| EP 0055045 | A | 30-06-1982 | AU | 543927 B | 09-05-1985 |
| | | | AU | 7866081 A | 24-06-1982 |
| | | | DK | 564581 A | 20-06-1982 |
| | | | JP | 1759047 C | 20-05-1993 |
| | | | JP | 4024339 B | 24-04-1992 |
| | | | JP | 57131733 A | 14-08-1982 |
| | | | ZA | 8108682 A | 26-01-1983 |
| EP 0537389 | A | 21-04-1993 | AUCUN | | |
| US 4741891 | A | 03-05-1988 | AU | 542052 B | 07-02-1985 |
| | | | AU | 7131481 A | 17-12-1981 |
| | | | GB | 2077709 A,B | 23-12-1981 |
| | | | US | 4876412 A | 24-10-1989 |
| | | | US | 4836996 A | 06-06-1989 |
| | | | US | 5098685 A | 24-03-1992 |
| EP 0051318 | A | 12-05-1982 | JP | 1443930 C | 08-06-1988 |
| | | | JP | 57145821 A | 09-09-1982 |
| | | | JP | 62048648 B | 15-10-1987 |
| | | | JP | 57203019 A | 13-12-1982 |
| | | | JP | 58013526 A | 26-01-1983 |
| | | | JP | 1447629 C | 30-06-1988 |
| | | | JP | 57095821 A | 14-06-1982 |
| | | | JP | 62057572 B | 01-12-1987 |
| | | | AU | 550092 B | 06-03-1986 |
| | | | AU | 7708881 A | 13-05-1982 |
| | | | CA | 1169038 A | 12-06-1984 |
| | | | US | 4695667 A | 22-09-1987 |
| WO 9616004 | A | 30-05-1996 | AU | 4737896 A | 17-06-1996 |
| | | | BR | 9509756 A | 16-06-1998 |
| | | | CA | 2206891 A | 30-05-1996 |
| | | | CN | 1170396 A | 14-01-1998 |
| | | | EP | 0793634 A | 10-09-1997 |
| | | | JP | 11501286 T | 02-02-1999 |

EPO FORM P0460

Pour tout renseignement concernant cette annexe : voir Journal Officiel de l'Office européen des brevets, No.12/82